# EUROPEAN PATENT SPECIFICATION

(11) **EP 3 194 616 B1**
(45) Date of publication and mention of the grant of the patent: **29.07.2020**
(21) Application number: 15766784.1
(22) Date of filing: 16.09.2015
(51) Int. Cl.: C12Q 1/6886, C12Q 1/6804

(54) **MRNA AND/OR PROTEIN OF ERCC1 ISOFORM 3 FOR USE IN DIAGNOSING A RESISTANCE AGAINST A THERAPEUTIC AGENT AND METHOD FOR DIAGNOSING A RESISTANCE AGAINST A THERAPEUTIC AGENT USING SAID MRNA AND/OR PROTEIN**
MRNA UND/ODER PROTEIN VON ERCC1-ISOFORM 3 ZUR VERWENDUNG BEI DER DIAGNOSE EINER RESISTENZ GEGEN EIN THERAPEUTIKUM UND VERFAHREN ZUR DIAGNOSE EINER RESISTENZ GEGEN EIN THERAPEUTIKUM UNTER VERWENDUNG DES BESAGTEN MRNA UND/ODER PROTEINS
MRNA ET/OU PROTÉINE DE ERCC1 ISOFORME 3 DESTINÉS À ÊTRE UTILISÉS POUR DIAGNOSTIQUER UNE RÉSISTANCE CONTRE UN AGENT THÉRAPEUTIQUE ET PROCÉDÉ PERMETTANT DE DIAGNOSTIQUER UNE RÉSISTANCE CONTRE UNE MRNA UTILISANT LEDIT AGENT THÉRAPEUTIQUE ET/OU DE PROTÉINE

(30) Priority: 18.09.2014 EP 14185247
(43) Date of publication of application: 26.07.2017
(73) Proprietor: AdnaGen GmbH, 30853 Hannover-Langenhagen (DE)
(72) Inventor: HAUCH, Siegfried, 30657 Isernhagen-Süd (DE); BOKELOH, Yvonne, 31688 Nienstädt (DE); WAGNER, Jenny, 30900 Wedemark (DE)
(74) Representative: Pfenning, Meinig & Partner mbB
(86) International application number: PCT/EP2015/071170
(87) International publication number: WO 2016/042009

(56) References cited:
- EP-A1- 2 233 589
- US-A1- 2014 170 659
- LUC FRIBOULET ET AL: "ERCC1 function in nuclear excision and interstrand crosslink repair pathways is mediated exclusively by the ERCC1-202 isoform", CELL CYCLE, vol. 12, no. 20, 15 October 2013 (2013-10-15), pages 3298-3306, XP055176453, ISSN: 1538-4101, DOI: 10.4161/cc.26309 cited in the application
- ZHANG QIANG ET AL: "A prospective study of biomarker-guided chemotherapy in patients with non-small cell lung cancer", CANCER CHEMOTHERAPY AND PHARMACOLOGY, SPRINGER VERLAG, BERLIN, vol. 74, no. 4, 14 August 2014 (2014-08-14), pages 839-846, XP035397537, ISSN: 0344-5704, DOI: 10.1007/S00280-014-2513-X [retrieved on 2014-08-14]

## Description

The detection of circulating tumor cells (CTC) is widely accepted as an independent prognostic tool in solid cancers as well in primary disease and metastatic disease. Therapy failure during treatment of said diseases was indicated by CTC not disappearing in the course of a given therapy. However, it turned out that such prognostic information alone is of limited usefulness since it does not help in predicting therapeutic success of certain therapeutic agents before a therapy is even started.

Thus, there is a need for further characterization of CTC in order to identify markers which are present in CTC and may give useful information about the possible success of a therapy with certain therapeutic agents, as well as information on putative therapeutic targets, where a future therapy can individually be adjusted to.

The AdnaTest®, which combines immunomagnetic capturing followed by a molecular characterization of such captured cells by means of mRNA profiling, is a useful tool to address such questions. For example, in ovarian cancer, an early identification of a successful therapy is of paramount importance, especially in ovarian cancers where prognosis is already bad. Such method is disclosed e.g. in EP 1 409 727 A2, which is herewith incorporated by reference.

Platinum-based therapeutic agents or regimens are the first and most promising choice in the treatment of some forms of cancer, e.g. ovarian cancer after surgery. However, about 20% of the patients already have developed, or are about to develop, resistance against platinum-based therapeutics. Thus, said patients no longer benefit from these therapeutics or these regimen.

ERCC1 is a DNA repair gene which was regarded for a long time as a tissue biomarker to provide predictive information about a resistance to platinum-based therapeutic agents. However, it has recently been found that immuno-histochemical detection of ERCC1 protein in tissue lacks specificity and was unsuitable for correct prediction of resistance to platinum-based therapeutic agents. It was found that ERCC1 isoforms 2, 3 and 4 are non-functional in nucleotide excision repair capacity. Consequently, it was assumed that they are unsuitable for use as a biomarker. Among the four different isoforms of ERCC1, only ERCC1 isoform 1 was found to be suitable to be used as a biomarker (see Friboulet L. et al., Cell Cycle, vol. 12, p. 3298-3306). However, ERCC1 isoforms 1 and 4 are highly similar which means that it is not possible to date to specifically detect isoform 1 expression without codetection of isoform 4 expression. Importantly, the expression of ERCC1 isoforms 1 and 4 is time and tissue-specific which means a detection of isoform 1 is afflicted with a large inter-tissue variation and is error-prone because of interference with isoform 4 codetection.

Friboulet, L. et al. (Cell Cycle, 2013, vol. 12, no. 20, p. 3298-3306) teaches that ERCC1 isoform-202 is the only ERCC1 isoform which functions in nuclear excision and interstrand crosslink repair pathways and is a useful biomarker for customizing anticancer therapies.

US 2014/170659 A1 discloses an in vitro method for detecting susceptibility of a tumor cell to a chemotherapy, wherein the method includes the step of measuring the expression level of the ERCC1 isoform-202.

EP 2 233 589 A1 discloses a diagnostic method for predicting the benefit of the response of a subject diagnosed with cancer to a platinum compound-based adjuvant chemotherapy.

Zhang, Q. et al. (Cancer Chemotherapy and Pharmacology, 2014, vol. 74, no. 4, p. 839-846) discloses a prospective study of biomarker-guided chemotherapy in patients with non-small cell lung cancer.

Starting herefrom, it was the object of the present invention to provide a biomarker which provides a reproducible and tissue-independent prediction of resistance against a therapeutic agent which is less prone to detection errors.

The problem is solved by the use of ERCC1 isoform 3 protein according to claim 1, the use of ERCC1 isoform 3 mRNA according to claim 2, the method for diagnosing a resistance against a therapeutic agent according to claim 3 and the use of ERCC1 isoform 3 protein and/or mRNA as biomarker according to claim 4. The dependent claims refer to preferred embodiments thereof.

According to the invention, an *in vitro*-use of ERCC1 isoform 3 protein and/or mRNA is suggested for diagnosing a resistance against a platinum-based cancer therapeutic agent. The isoform 3 of ERCC1 is also known as "ERCC1 201" and the protein has the identifier P07992-3 at the "Swiss-Prot" protein database (see www.uniprot.org). With ERCC1 isoform 3 mRNA, any mRNA is understood which codes for the ERCC1 isoform 3 protein having the identifier P07992-3 at the "Swiss-Prot" protein database.

It was surprisingly found that the level of ERCC1 isoform 3 protein production in a cell, as well as the level of ERCC1 isoform 3 mRNA expression in a cell, especially in circulating tumor cells, correlates with resistance to therapeutic agents like platinum-based therapeutic agents. The finding is surprising because the isoform 3 of ERCC1 is known not to be involved in the development of resistance to therapeutic agents. The advantage of ERCC1 isoform 3 protein and/or mRNA is that it is structurally considerably different to the other isoforms of ERCC1 and thus can be determined without the problem of codetection of isoforms 1, 2 and/or 4. Consequently, ERCC1 isoform 3 is a more reliable marker for detecting resistances compared to e.g. ERCC1 isoform 1.

Additionally, a method for diagnosing a resistance against a platinum-based cancer therapeutic agent is provided. The method comprises or consists of the steps of
a) measuring an amount of ERCC1 isoform 3 protein and/or an amount of ERCC1 isoform 3 mRNA, which present in a liquid sample or a tissue sample, preferably a liquid sample from a human body or a tissue sample from a human body, and
b) comparing the measured amount to a predetermined value, wherein a measured amount above the predetermined value indicates resistance against said therapeutic agent.

According to the invention, the therapeutic agent is a platinum-based cancer therapeutic agent.

Additionally, the use of ERCC1 isoform 3 protein and/or ERCC1 isoform 3 mRNA as biomarker is suggested, wherein the biomarker is a biomarker used for detecting a resistance to a platinum-based cancer therapeutic agent.

Regarding the above recited protein, mRNA, method and/or use, the following embodiments are particularly preferred.

Diagnosing may be done on a liquid sample from a human body and/or a tissue sample from a human body, preferably an isolated liquid sample from a human body and/or a tissue sample from a human body (*in*-*vitro*-method). The sample may comprise or consist of a body fluid (e.g. peripheral blood, sputum, ascites, lymph, urine and/or bone marrow). The sample may also comprise or consist of a biopsy material (e.g. a biopsy material from a primary tumor).

The cancer may be selected from the group comprising or consisting of a solid cancer, preferably ovarian cancer, breast cancer, lung cancer, prostate cancer, pancreatic cancer, bladder cancer, gastric cancer, colon cancer, testicular cancer and neck cancer. Particularly preferred is ovarian cancer. Most preferably diagnosing regards ovarian cancer after surgery.

Diagnosing the resistance may comprise a step of isolating circulating tumor cells, preferably by a multi-antibody capturing method, and a step of determining the level of expression of ERCC1 isoform 3 protein and/or ERCC1 isoform 3 mRNA in the isolated circulating tumor cells. In this regard, the isolation of the circulating tumor cells may be performed on a liquid sample from a human body and/or a tissue sample from a human body. Preferably, the isolation of the circulating tumor cells is performed on an isolated liquid sample from a human body and/or a tissue sample from a human body (*in-vitro-*method).

For example, for isolating circulating tumor cells, the AdnaTest® OvarianCancerSelect / Detect may be used.

The step of isolating circulating tumor cells may comprise or consist of the steps of
a) mixing a liquid sample, preferably a liquid sample from a human body, and/or a tissue sample from a human body, containing circulating tumor cells, with a predetermined combination of at least two antibodies and/or antibody derivatives, wherein the antibodies and/or antibody derivatives have a binding affinity to circulating tumor cells; and
b) isolating complexes between i) the antibodies and/or antibody derivatives and ii) the circulating tumor cells, from the sample.

The antibodies and/or antibody derivatives are preferably coupled to at least one magnetic or paramagnetic particle. In this case, magnetic force may be used for isolation of said complexes. Additionally or alternatively, the antibodies and/or antibody derivatives may be coupled to at least one fluorescent molecule. In this case, fluorescence activated cell sorting can be used for isolation of said complexes.

At least one of the two antibodies and/or antibody derivatives may be selected from the group comprising or consisting of GP1.4, MOC-31, Ber-EP-4, HMPV.2, HMEIV.2, 8B6, E29, 10E9 (anti-HER2), 2D3 (anti-EpCAM) anti-cMET, anti-EGFR, anti-cKIT, anti-IGFR and 131-11741.

Diagnosing the resistance may comprise the step of
a) lysing isolated circulating tumor cells,
b) measuring an amount of ERCC1 isoform 3 protein and/or ERCC1 isoform 3 mRNA, present in the lysed isolated circulating tumor cells, and
c) measuring an amount of at least one mRNA which is present in the lysed isolated circulating tumor cells and is different to ERCC1 isoform 3 mRNA, wherein the at least one mRNA is selected from the group comprising or consisting of mRNAs which are markers for cells of epithelial, mesenchymal or stem cell phenotype,
wherein step b) and step c) may be changed in their sequence.

Preferably, measuring in step b) and/or step c) is done by RT-PCR, LCR, NASBA, a hybridisation method and/or a method containing the steps of PCR, digesting of the PCR product with restriction enzymes to fragments and analysis of said obtained fragments.

The at least one mRNA, which is different to ERCC1 isoform 3 mRNA, may be selected from the group comprising or consisting of mRNAs which are markers for cells of epithelial, mesenchymal or stem cell phenotype, preferably the group comprising or consisting of mRNAs recited in Table 1.

**Table 1**

| **epithelial markers** | **mesenchymal markers** | **stem cell markers** |
|---|---|---|
| CD166 | 5'-Nucleotidase/CD73 | BMI1 |
| Adenosine 5'-Triphosphatase | ALCAM/CD166 | ALDH1 |
| CD138 (Syndecan-1) | Aminopeptidase N/CD13 | CD44 |
| beta-Crystallin | BMPR-IA/ALK-3 | CD24 |
| beta-Defensin 2 | BMPR-IB/ALK-6 | KRT19 |
| beta-Defensin 3 | BMPR-II | BRCA1 |
| BRCA1 | N-Cadherin | PTEN |
| BTEB1 | CD44 | MSI1 |
| Calcitonin Gene-Related Peptide (CGRP) | CD45 | CD34 |
| Calcyclin | CD90/Thyl | OCT-4 |
| Carcinoembryonic Antigen (CEA) | CD117/c-kit | SSEA |
| Cathepsin E (CaE) | CD45RO | CD133 |
| Caveolin-1 | Endoglin/CD105 | ABCG-2 |
| CD138 (Syndecan-1) | Fibronectin | SCA1 |
| CD151 | Fibronectin/Anastellin | STRO-1 |
| CD46 | HLA Class I | Nestin |
| Connexin-43 (Cx43) | ICAM-1/CD54 | PSA-NCAM |
| Muc-1 | Integrin alpha 1/CD49a | p75 Neurotropin |
| EMA | Integrin alpha 5/CD49e | N-Cadherin |
| Epithelial Sodium Channel-a | Inteprin alpha V/CD51 | HLA Class1 |
| Epithelial Sodium Channel-P | Integrin beta 1/CD29 | CXCR4 |
| Epithelial Sodium Channel-γ | NCAM-1/CD56 | CSPG4 |
| Epithelial Sodium Channel-δ | NGF R/TNFRSF16 | CD38 |
| Epithelium specific antigen (EP-CAM, ESA) (AUA1) | Nucleostemin | CD90 |
| Epithelium/endothelial cells [PCX, Podocalyxin] | PDGF R alpha | CD117 |
| Exo-1 (Pa-G14) | Sca-1/Ly6 | CD146 |
| EZH2 | SSEA-4 | |
| Ezrin | STRO-1 | |
| Fas Ligand/TNFSF6 | TfR (Transferrin R) | |
| Fibrinogen (1F3) | VCAM-1/CD106 | |
| Foxa1 | Vimentin | |
| GABRP | Pi3KCA | |
| Galectin-3 | Snail | |
| Pan-Cytokeratin (AE1/AE3) | Twist | |
| Pan-Cytokeratin (KL1) | Slug | |
| GGT (gamma-glutamyl transpeptidase) | SMAD2/3 | |
| Glutamine Synthetase | AKt2 | |
| Heat Shock Protein 27 [HSP27] | cKit | |
| HLA-DR | cMet | |
| Lactoferrin | Notch | |
| LAMP-1 (lysosomal-associated membrane proprotein 1) | Sip1 | |
| Lectin | FOXC2 | |
| Lectins | SOX10 | |
| Leu-7 | beta Catenin | |
| MMR | VEGF | |
| MOC-31 | MMP2,3,9 | |
| NCAM-L1 (neural cell adhesion molecule L1) | mTOR | |
| Nectin-2/CD112 | cMet | |
| Normal Epithelial Cell Specific-1 (NES1)/kallikrein-10 | HER2/NEU | |
| NSE (neuron-specific enolase) | EGFR | |
| Ovarian Cancer Antigen [CA125] | MAGE3 | |
| p16 (INK4A) | | |
| P2X7 | | |
| p63 | | |
| P-Cadherin | | |
| plgR | | |
| Prominin-1 (CD133) | | |
| Prostasin/Prss8 | | |
| PSA | | |
| Prostatic Binding Protein (PBP) | | |
| Prosurfactant Protein B | | |
| Prosurfactant Protein C | | |
| PSCA (Prostate stem cell antigen) | | |
| Rab13 | | |
| RAGE | | |
| Rex-1 (zinc-finger protein-42, Zfp42) | | |
| Secretory Component (SC) | | |
| Sucrase-isomaltase (SI) | | |
| Surfactant Protein A | | |
| Surfactant Protein B | | |
| Surfactant protein C (SPC) | | |
| Surfactant Protein D | | |
| Survivin | | |
| TfR (Transferrin Rezetor) | | |
| Transthyretin | | |
| UGRP1/SCGB3A2 | | |
| VAT-1 | | |
| PSMA | | |
| Cystatin C | | |
| Cytokeratin 10 | | |
| Cytokeratin 12 | | |
| Cytokeratin 13 | | |
| Cytokeratin 1-3 | | |
| Cytokeratin 14 | | |
| Cytokeratin 15 | | |
| Cytokeratin 16 | | |
| Cytokeratin 17 | | |
| Cytokeratin 18 | | |
| Cytokeratin 19 | | |
| Cytokeratin 20 | | |
| Cytokeratin 3 | | |
| Cytokeratin 4 | | |
| Cytokeratin 5 | | |
| Cytokeratin 5, 6 | | |
| Cytokeratin 7 | | |
| Cytokeratin 8 | | |
| Cytokeratin 8, 14, 18, 19 | | |
| Cytokeratin 8, 18 | | |
| Cytokeratin AE1 | | |
| Cytokeratin AE3 | | |
| Desmin | | |
| Desmocollin-2 | | |
| Desmocollin-3 | | |
| E-Cadherin | | |
| Ber-EP4 | | |
| Claudin-7 | | |

Diagnosing the resistance preferably comprises a step of comparing a measured expression level of ERCC1 isoform 3 protein and/or ERCC1 isoform 3 mRNA with a first predetermined value, wherein a measured expression level of ERCC1 isoform 3 protein and/or ERCC1 isoform 3 mRNA above the first predetermined value indicates a resistance against said therapeutic agent.

Diagnosing the resistance may comprise the steps of
a) comparing a measured expression level of ERCC1 isoform 3 protein and/or ERCC1 isoform 3 mRNA with a first predetermined value; and
b) comparing a measured expression level of at least one mRNA, which is different to ERCC1 isoform 3 mRNA, with a second predetermined value, wherein the at least one mRNA is selected from the group comprising or consisting of mRNAs which are markers for cells of epithelial, mesenchymal or stem cell phenotype, and
wherein a measured expression level of ERCC1 isoform 3 protein and/or ERCC1 isoform 3 mRNA above the first predetermined value and a measured expression level of said at least one mRNA, which is different to ERCC1 isoform 3 mRNA, above the second predetermined value indicates a resistance against said therapeutic agent.

The at least one mRNA, which is different to ERCC1 isoform 3, may be selected from the group comprising or consisting of mRNAs recited in Table 1 (see above).

With reference to the following examples and figures, the subject-matter according to the invention is intended to be explained in more detail without wishing to restrict said subject-matter to the special embodiments shown here.

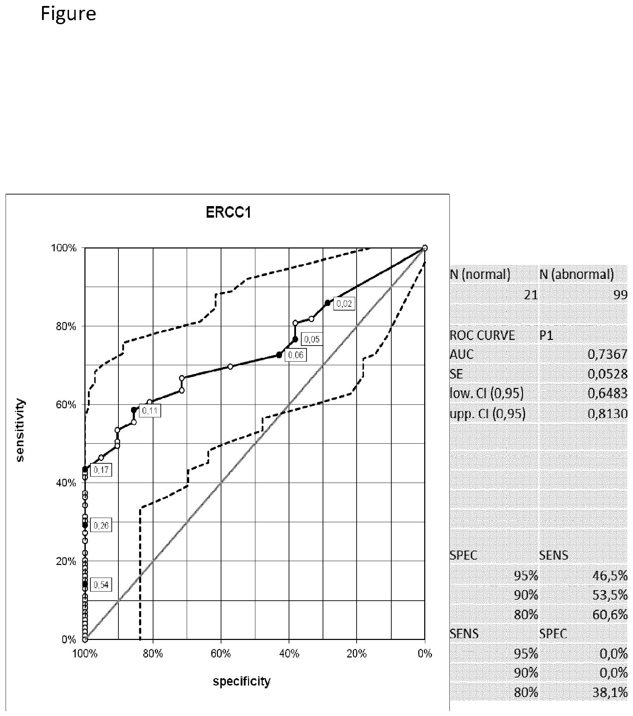

The Figure shows the results of the PCR assay validation by ROC curve analysis. Plotted is the sensitivity against the specificity of ERCC1 isoform 3 detection among all ERCC1 isoforms. More than 90% specificity (at 50% sensitivity) is obtained at a fragment concentration of approx. 0.2 ng/µl.

### Example 1 - PCR assay validation by ROC curve analysis

In the ROC curve analysis, the cut-off value corresponding to the specificity of the detection of ERCC1 isoform 3 by semi-quantitative PCR was determined.

cDNA was extracted from blood samples of 99 ovarian cancer patients and 21 healthy patients using the AdnaTest OvarianCancerSelect/Detect assay kit. Said cDNA potentially comprises all four isoforms of ERCC1. Subsequently, a fragment concentration analysis regarding the level of ERCC1 isoform 3 was performed using an Agilent 2100 Bioanalyzer (Agilent Technologies).

At a fragment concentration of approx. 0.2 ng/µl, a specificity for detection of ERCC1 isoform 3 among all ERCC1 isoforms of more than 90% was reached (see Figure).

In conclusion, PCR (e.g. semi-quantitative PCR) is a suitable method amongst others to specifically detect ERCC1 isoform 3 among all ERCC1 isoforms.

### Example 2 - Prediction of platinum resistance by ERCC1 isoform 3

In a clinical trial, blood samples of 143 pre-surgery ovarian cancer patients were analysed for the presence of circulationg tumor cells (CTC) using the AdnaTest OvarianCancerSelect/Detect assay kit. Subsequently, all 143 patients received surgery followed by platinum-based chemotherapy.

Analysis of CTC was performed by immunomagnetic CTC enrichment targeting the epitopes of epithelial cell adhesion molecule (EPCAM) (also known as GA733-2) and mucin 1, cell surface associated (MUC1) and subsequently conducting a multiplex reverse-transcription PCR to detect the transcripts EPCAM, MUC1, and mucin 16, cell surface associated (MUC16) (also known as CA125), including, in a separate approach, ERCC1 isoform 3 transcripts.

The presence of CTC was observed in 14 % of the patient samples. In said 14 % CTC positive patient samples, ERCC1 isoform 3 expression was found to be positive in 57 % of the analysed samples. Thus, in relation to all of the 143 analysed patient samples (i.e. in relation to all 143 patients), ERCC1 isoform 3-positive CTC (CTC ERCC1+) were observed in 8 % of the samples (i.e. in 8 % of all the 143 patients).

After the 143 patients received platinum-based chemotherapy, a multivariate log regression analysis was performed. ERCC1 isoform 3-positive CTC (CTC ERCC1+) were identified as independent predictive factor for platinum resistance as well as an independent prognostic factor for disease-free survival (DFS) (P = 0.012 and P = 0.007, respectively). The results of the analysis are shown in the Table 2 below.

**Table 2: "ERCC1" refers to ERCC1 isoform 3, "DFS" refers to disease-free survival, "OS" refers to overall survival, "HR" refers to hazard ratio, "Figo" refers to the clinical Figo staging, "pN" refers to lymph node stage, "M" refers to metastatic stage, "G" refers to Grading, "RTB" refers to "remaining tumor burden", "CTC pre" refers to circulating tumor cells before therapy, "CTC post" refers to circulating tumor cells after therapy, "CTC (ERCC1+)" refers to CTC which overexpress ERCC1 isoform 3 mRNA.**

| Cox Regression | DFS | | OS | | Platinum resistance (log. Regression) | |
|---|---|---|---|---|---|---|
| | Independent | HR | Independent | HR | Independent | HR |
| Figo | <0,0005 | 10,5 | 0,02 | 3,6 | <0,0005 | 10,5 |
| pN | * | * | * | * | * | * |
| M | <0,0005 | 3,9 | 0,026 | 2,3 | <0,0005 | 3,91 |
| G | <0,0005 | 0,29 | * | * | <0,0005 | 0,29 |
| RTB | * | * | <0,0005 | 2,0-7,9 | * | * |
| CTC pre | * | * | * | * | * | * |
| CTC post | * | * | * | * | * | * |
| CTC (ERCC1+) | 0,007 | 3,6 | * | * | 0,0012 | 3,58 |

In conclusion, at primary diagnosis of ovarian cancer, the presence of ERCC1 isoform 3 in CTC can serve as a blood-based diagnostic biomarker for predicting platinum resistance as well as for providing a prognosis for a disease-free survival.

## Claims

1. *In vitro*-use of ERCC1 isoform 3 protein in diagnosing a resistance against a platinum-based cancer therapeutic agent.

2. *In vitro*-use of ERCC1 isoform 3 mRNA in diagnosing a resistance against a platinum-based cancer therapeutic agent.

3. Method for diagnosing a resistance against a platinum-based cancer therapeutic agent, comprising or consisting of the steps of
a) measuring an amount of ERCC1 isoform 3 protein and/or an amount of ERCC1 isoform 3 mRNA, which present in a liquid sample or a tissue sample, and
b) comparing the measured amount to a predetermined value, wherein an amount above the predetermined value indicates resistance against said therapeutic agent.

4. Use of ERCC1 isoform 3 protein and/or ERCC1 isoform 3 mRNA as a biomarker, wherein the biomarker is a biomarker used for detecting a resistance to a platinum-based cancer therapeutic agent.

5. Use of ERCC1 isoform 3 protein according to claim 1, use of ERCC1 isoform 3 mRNA according to claim 2, method according to claim 3 or use according to claim 4, **characterized in that** the diagnosing is done on a liquid sample from a human body and/or a tissue sample from a human body, preferably an isolated liquid sample from a human body and/or an isolated tissue sample from a human body.

6. Use of ERCC1 isoform 3 protein according to one of claims 1 and 5, use of ERCC1 isoform 3 mRNA according to one of claims 2 and 5, method according to one of claims 3 and 5 or use according to one of claims 4 and 5, **characterized in that** the sample comprises or consists of a body fluid, e.g. peripheral blood, sputum, ascites, lymph, urine and/or bone marrow, and/or a biopsy material, e.g. from a primary tumor.

7. Use of ERCC1 isoform 3 protein according to one of claims 1, 5 and 6, use of ERCC1 isoform 3 mRNA according to one of claims 2, 5 and 6, method according to one of claims 3, 5 and 6 or use according to one of claims 4 to 6, **characterized in that** the cancer is selected from the group comprising or consisting of a solid cancer, preferably ovarian cancer, breast cancer, lung cancer, prostate cancer, pancreatic cancer, bladder cancer, gastric cancer, colon cancer, testicular cancer and neck cancer.

8. Use of ERCC1 isoform 3 protein according to one of claims 1 and 5 to 7, use of ERCC1 isoform 3 mRNA according to one of claims 2 and 5 to 7, method according to one of claims 3 and 5 to 7 or use according to one of claims 4 to 7, **characterized in that** diagnosing the resistance comprises, on a liquid sample from a human body, a step of isolating circulating tumor cells, preferably by a multi-antibody capturing method, and a step of determining the level of expression of ERCC1 isoform 3 protein and/or ERCC1 isoform 3 mRNA in the isolated circulating tumor cells.

9. Use of ERCC1 isoform 3 protein according to claim 8, use of ERCC1 isoform 3 mRNA according to claim 8, method according to claim 8 or use according to claim 8, **characterized in that** the step of isolating circulating tumor cells comprises or consists of the steps of
a) mixing a liquid sample with a predetermined combination of at least two antibodies and/or antibody derivatives having binding affinity to circulating tumor cells; and
b) isolating complexes between i) the antibodies and/or antibody derivatives and ii) the circulating tumor cells, from the sample.

10. Use of ERCC1 isoform 3 protein according to claim 9, use of ERCC1 isoform 3 mRNA according to claim 9, method according to claim 9 or use according to claim 9, **characterized in that** said antibodies and/or antibody derivatives are coupled to
a) at least one magnetic or paramagnetic particle and preferably magnetic force is used for isolation of said complexes; and/or
b) at least one fluorescent molecule and preferably fluorescence activated cell sorting is used for isolation of said complexes.

11. Use of ERCC1 isoform 3 protein according to one of claims 9 and 10, use of ERCC1 isoform 3 mRNA according to one of claims 9 and 10, method according to one of claims 9 and 10 or use according to one of claims 9 and 10, **characterized in that** at least one of the two antibodes and/or antibody derivatives is selected from the group comprising or consisting of GP1.4, MOC-31, Ber-EP-4, HMPV.2, HMEIV.2, 8B6, E29, 10E9 (anti-HER2), 2D3 (anti-EpCAM) anti-cMET, anti-EGFR, anti-cKIT, anti-IGFR and 131-11741.

12. Use of ERCC1 isoform 3 protein according to one of claims 8 to 11, use of ERCC1 isoform 3 mRNA according to one of claims 8 to 11, method according to one of claims 8 to 11 or use according to one of claims 8 to 11, **characterized in that** diagnosing the resistance comprises the step of
a) lysing said isolated circulating tumor cells,
b) measuring the amount of ERCC1 isoform 3 protein and/or ERCC1 isoform 3 mRNA, present in the lysed isolated circulating tumor cells, and
c) measuring the amount of at least one mRNA, which is present in the lysed isolated circulating tumor cells and is different to ERCC1 isoform 3 mRNA, wherein the at least one mRNA is selected from the group comprising or consisting of mRNAs which are markers for cells of epithelial, mesenchymal or stem cell phenotype,
wherein step b) and step c) may be changed in their sequence, and
wherein measuring in step b) and/or step c) is preferably done by RT-PCR, LCR, NASBA, a hybridisation method and/or a method containing the steps of PCR, digesting of the PCR product with restriction enzymes to fragments and analysis of said obtained fragments.

13. Use of ERCC1 isoform 3 protein according to one of claims 1 and 5 to 12, use of ERCC1 isoform 3 mRNA according to one of claims 2 and 5 to 12, method according to one of claims 3 and 5 to 12 or use according to one of claims 4 to 12, **characterized in that** diagnosing the resistance comprises a step of comparing a measured expression level of ERCC1 isoform 3 protein and/or ERCC1 isoform 3 mRNA with a first predetermined value, wherein a measured expression level of ERCC1 isoform 3 protein and/or ERCC1 isoform 3 mRNA above the first predetermined value indicates a resistance against said therapeutic agent.

14. Use of ERCC1 isoform 3 protein according to claim 13, use of ERCC1 isoform 3 mRNA according to claim 13, method according to claim 13 or use according to claim 13, **characterized in that** diagnosing the resistance comprises the steps of
a) comparing a measured expression level of ERCC1 isoform 3 protein and/or ERCC1 isoform 3 mRNA with a first predetermined value; and
b) comparing a measured expression level of at least one mRNA, which is different to ERCC1 isoform 3 mRNA, with a second predetermined value, wherein the at least one mRNA is selected from the group comprising or consisting of mRNAs which are markers for cells of epithelial, mesenchymal or stem cell phenotype, and
wherein a measured expression level of said ERCC1 isoform 3 protein and/or said ERCC1 isoform 3 mRNA above the first predetermined value and a measured expression level of said at least one mRNA, which is different to ERCC1 isoform 3 mRNA, above the second predetermined value indicates a resistance against said therapeutic agent.

## Patentansprüche

1. *In-vitro*-Verwendung von ERCC1-Isoform-3-Protein beim Diagnostizieren einer Resistenz gegen ein platinbasiertes Krebstherapeutikum.

2. *In-vitro*-Verwendung von ERCC1-Isoform-3-mRNA beim Diagnostizieren einer Resistenz gegen ein platinbasiertes Krebstherapeutikum.

3. Verfahren zum Diagnostizieren einer Resistenz gegen ein platinbasiertes Krebstherapeutikum, umfassend oder bestehend aus den Schritten des
a) Messens einer Menge von ERCC1-Isoform-3-Protein und/oder einer Menge von ERCC1-Isoform-3-mRNA, die in einer Flüssigkeitsprobe oder einer Gewebeprobe vorhanden ist, und
b) Vergleichens der gemessenen Menge mit einem vorherbestimmten Wert, wobei eine Menge über dem vorherbestimmten Wert eine Resistenz gegen das Therapeutikum anzeigt.

4. Verwendung von ERCC1-Isoform-3-Protein und/oder ERCC1-Isoform-3-mRNA als einen Biomarker, wobei der Biomarker ein Biomarker ist, der zum Nachweisen einer Resistenz gegen ein platinbasiertes Krebstherapeutikum verwendet wird.

5. Verwendung von ERCC1-Isoform-3-Protein nach Anspruch 1, Verwendung von ERCC1-Isoform-3-mRNA nach Anspruch 2, Verfahren nach Anspruch 3 oder Verwendung nach Anspruch 4, **dadurch gekennzeichnet, dass** das Diagnostizieren an einer Flüssigkeitsprobe von einem menschlichen Körper und/oder einer Gewebeprobe von einem menschlichen Körper, vorzugsweise einer isolierten Flüssigkeitsprobe von einem menschlichen Körper und/oder einer isolierten Gewebeprobe von einem menschlichen Körper, durchgeführt wird.

6. Verwendung von ERCC1-Isoform-3-Protein nach einem der Ansprüche 1 und 5, Verwendung von ERCC1-Isoform-3-mRNA nach einem der Ansprüche 2 und 5, Verfahren nach einem der Ansprüche 3 und 5 oder Verwendung nach einem der Ansprüche 4 und 5, **dadurch gekennzeichnet, dass** die Probe eine Körperflüssigkeit, z.B. peripheres Blut, Sputum, Bauchwasser, Lymphe, Urin und/oder Knochenmark, und/oder ein Biopsiematerial, z.B. aus einem Primärtumor, enthält oder daraus besteht.

7. Verwendung von ERCC1-Isoform-3-Protein nach einem der Ansprüche 1, 5 und 6, Verwendung von ERCC1-Isoform-3-mRNA nach einem der Ansprüche 2, 5 und 6, Verfahren nach einem der Ansprüche 3, 5 und 6 oder Verwendung nach einem der Ansprüche 4 bis 6, **dadurch gekennzeichnet, dass** der Krebs aus der Gruppe ausgewählt ist, die einen soliden Krebs, vorzugsweise Eierstockkrebs, Brustkrebs, Lungenkrebs, Prostatakrebs, Bauchspeicheldrüsenkrebs, Blasenkrebs, Magenkrebs, Dickdarmkrebs, Hodenkrebs und Halskrebs, umfasst oder daraus besteht.

8. Verwendung von ERCC1-Isoform-3-Protein nach einem der Ansprüche 1 und 5 bis 7, Verwendung von ERCC1-Isoform-3-mRNA nach einem der Ansprüche 2 und 5 bis 7, Verfahren nach einem der Ansprüche 3 und 5 bis 7 oder Verwendung nach einem der Ansprüche 4 bis 7, **dadurch gekennzeichnet, dass** das Diagnostizieren der Resistenz, an einer Flüssigkeitsprobe von einem menschlichen Körper, einen Schritt des Isolierens zirkulierender Tumorzellen, vorzugsweise durch ein Multi-Antikörper-Erfassungsverfahren, und einen Schritt des Bestimmens des Expressionsniveaus von ERCC1-Isoform-3-Protein und/oder ERCC1-Isoform-3-mRNA in den isolierten zirkulierenden Tumorzellen, umfasst.

9. Verwendung von ERCC1-Isoform-3-Protein nach Anspruch 8, Verwendung von ERCC1-Isoform-3-mRNA nach Anspruch 8, Verfahren nach Anspruch 8 oder Verwendung nach Anspruch 8, **dadurch gekennzeichnet, dass** der Schritt des Isolierens zirkulierender Tumorzellen umfasst oder besteht aus den Schritten des
a) Mischens einer Flüssigkeitsprobe mit einer vorherbestimmten Kombination von mindestens zwei Antikörpern und/oder Antikörperderivaten, die eine Bindungsaffinität zu zirkulierenden Tumorzellen haben; und
b) Isolierens aus der Probe von Komplexen zwischen i) den Antikörpern und/oder Antikörperderivaten und ii) den zirkulierenden Tumorzellen.

10. Verwendung von ERCC1-Isoform-3-Protein nach Anspruch 9, Verwendung von ERCC1-Isoform-3-mRNA nach Anspruch 9, Verfahren nach Anspruch 9 oder Verwendung nach Anspruch 9, **dadurch gekennzeichnet, dass** die Antikörper und/oder Antikörperderivate gekoppelt sind an
a) mindestens ein magnetisches oder paramagnetisches Teilchen und vorzugsweise Magnetkraft zur Isolierung der Komplexe verwendet wird; und/oder
b) mindestens ein fluoreszierendes Molekül und vorzugsweise eine fluoreszenzaktivierte Zellsortierung zur Isolierung der Komplexe verwendet wird.

11. Verwendung von ERCC1-Isoform-3-Protein nach einem der Ansprüche 9 und 10, Verwendung von ERCC1-Isoform-3-mRNA nach einem der Ansprüche 9 und 10, Verfahren nach einem der Ansprüche 9 und 10 oder Verwendung nach einem der Ansprüche 9 und 10, **dadurch gekennzeichnet, dass** mindestens einer der zwei Antikörper und/oder Antikörperderivate aus der Gruppe ausgewählt ist, die GP1.4, MOC-31, Ber-EP-4, HMPV.2, HMEIV.2, 8B6, E29, 10E9 (anti-HER2), 2D3 (anti-EpCAM), anti-cMET, anti-EGFR, anti-cKIT, anti-IGFR und 131-11741 enthält oder daraus besteht.

12. Verwendung von ERCC1-Isoform-3-Protein nach einem der Ansprüche 8 bis 11, Verwendung von ERCC1-Isoform-3-mRNA nach einem der Ansprüche 8 bis 11, Verfahren nach einem der Ansprüche 8 bis 11 oder Verwendung nach einem der Ansprüche 8 bis 11, **dadurch gekennzeichnet, dass** das Diagnostizieren der Resistenz den Schritt umfasst des
a) Lysierens der isolierten zirkulierenden Tumorzellen,
b) Messens der Menge von ERCC1-Isoform-3-Protein und/oder ERCC1-Isoform-3-mRNA, die in den lysierten isolierten zirkulierenden Tumorzellen vorhanden ist, und
c) Messens der Menge von mindestens einer mRNA, die in den lysierten isolierten zirkulierenden Tumorzellen vorhanden ist und sich von ERCC1-Isoform-3-mRNA unterscheidet, wobei die mindestens eine mRNA aus der Gruppe ausgewählt ist, die mRNAs enthält oder daraus besteht, die Marker für Zellen des epithelialen, mesenchymalen oder Stammzell-Phänotyps sind,
wobei Schritt b) und Schritt c) in ihrer Reihenfolge geändert werden können, und
wobei das Messen in Schritt b) und/oder Schritt c) vorzugsweise durch RT-PCR, LCR, NASBA, ein Hybridisierungsverfahren und/oder ein Verfahren, das die Schritte PCR, Verdau des PCR-Produkts mit Restriktionsenzymen zu Fragmenten und Analyse der erhaltenen Fragmente umfasst, durchgeführt wird.

13. Verwendung von ERCC1-Isoform-3-Protein nach einem der Ansprüche 1 und 5 bis 12, Verwendung von ERCC1-Isoform-3-mRNA nach einem der Ansprüche 2 und 5 bis 12, Verfahren nach einem der Ansprüche 3 und 5 bis 12 oder Verwendung nach einem der Ansprüche 4 bis 12, **dadurch gekennzeichnet, dass** das Diagnostizieren der Resistenz einen Schritt des Vergleichens eines gemessenen Expressionsniveaus von ERCC1-Isoform-3-Protein und/oder ERCC1-Isoform-3-mRNA mit einem ersten vorherbestimmten Wert umfasst, wobei ein gemessenes Expressionsniveau von ERCC1-Isoform-3-Protein und/oder ERCC1-Isoform-3-mRNA oberhalb des ersten vorherbestimmten Wertes eine Resistenz gegen das Therapeutikum anzeigt.

14. Verwendung von ERCC1-Isoform-3-Protein nach Anspruch 13, Verwendung von ERCC1-Isoform-3-mRNA nach Anspruch 13, Verfahren nach Anspruch 13 oder Verwendung nach Anspruch 13, **dadurch gekennzeichnet, dass** das Diagnostizieren der Resistenz die Schritte umfasst des
a) Vergleichens eines gemessenen Expressionsniveaus von ERCC1-Isoform-3-Protein und/oder ERCC1-Isoform-3-mRNA mit einem ersten vorherbestimmten Wert; und
b) Vergleichens eines gemessenen Expressionsniveaus von mindestens einer mRNA, die sich von ERCC1-Isoform-3-mRNA unterscheidet, mit einem zweiten vorherbestimmten Wert, wobei die mindestens eine mRNA aus der Gruppe ausgewählt ist, die mRNAs enthält oder daraus besteht, die Marker für Zellen des epithelialen, mesenchymalen oder Stammzell-Phänotyps sind, und
wobei ein gemessenes Expressionsniveau des ERCC1-Isoform-3-Proteins und/oder der ERCC1-Isoform-3-mRNA über dem ersten vorherbestimmten Wert und ein gemessenes Expressionsniveau der mindestens einen mRNA, die sich von ERCC1-Isoform-3-mRNA unterscheidet, über dem zweiten vorherbestimmten Wert, eine Resistenz gegen das Therapeutikum anzeigt.

## Revendications

1. Utilisation *in vitro* de la protéine de l'isoforme 3 d'ERCC1 dans le diagnostic d'une résistance à un agent thérapeutique du cancer à base de platine.

2. Utilisation *in vitro* de l'ARNm de l'isoforme 3 d'ERCC1 dans le diagnostic d'une résistance à un agent thérapeutique du cancer à base de platine.

3. Procédé de diagnostic d'une résistance à un agent thérapeutique du cancer à base de platine, comprenant ou consistant en les étapes consistant à
a) mesurer une quantité de la protéine de l'isoforme 3 d'ERCC1 et/ou une quantité de l'ARNm de l'isoforme 3 d'ERCC1, qui est présente dans un échantillon de liquide ou un échantillon de tissu, et
b) comparer la quantité mesurée à une valeur prédéterminée, où une quantité supérieure à la valeur prédéterminée indique une résistance audit agent thérapeutique.

4. Utilisation de la protéine de l'isoforme 3 d'ERCC1 et/ou de l'ARNm de l'isoforme 3 d'ERCC1 en tant que biomarqueur, où le biomarqueur est un biomarqueur utilisé pour détecter une résistance à un agent thérapeutique du cancer à base de platine.

5. Utilisation de la protéine de l'isoforme 3 d'ERCC1 selon la revendication 1, utilisation de l'ARNm de l'isoforme 3 d'ERCC1 selon la revendication 2, procédé selon la revendication 3 ou utilisation selon la revendication 4, caractérisé(e) en ce que le diagnostic est réalisé sur un échantillon de liquide d'un organisme humain et/ou un échantillon de tissu d'un organisme humain, de préférence un échantillon de liquide isolé d'un organisme humain et/ou un échantillon de tissu isolé d'un organisme humain.

6. Utilisation de la protéine de l'isoforme 3 d'ERCC1 selon l'une des revendications 1 et 5, utilisation de l'ARNm de l'isoforme 3 d'ERCC1 selon l'une des revendications 2 et 5, procédé selon l'une des revendications 3 et 5 ou utilisation selon l'une des revendications 4 et 5, caractérisé(e) en ce que l'échantillon comprend ou consiste en un liquide organique, par exemple du sang périphérique, des expectorations, des ascites, de la lymphe, de l'urine et/ou de la moelle osseuse, et/ou un matériel de biopsie, par exemple d'une tumeur primaire.

7. Utilisation de la protéine de l'isoforme 3 d'ERCC1 selon l'une des revendications 1, 5 et 6, utilisation de l'ARNm de l'isoforme 3 d'ERCC1 selon l'une des revendications 2, 5 et 6, procédé selon l'une des revendications 3, 5 et 6 ou utilisation selon l'une des revendications 4 à 6, caractérisé(e) en ce que le cancer est sélectionné dans le groupe comprenant ou consistant en un cancer solide, de préférence le cancer de l'ovaire, le cancer du sein, le cancer du poumon, le cancer de la prostate, le cancer du pancréas, le cancer de la vessie, le cancer de l'estomac, le cancer du côlon, le cancer du testicule et le cancer du cou.

8. Utilisation de la protéine de l'isoforme 3 d'ERCC1 selon l'une des revendications 1 et 5 à 7, utilisation de l'ARNm de l'isoforme 3 d'ERCC1 selon l'une des revendications 2 et 5 à 7, procédé selon l'une des revendications 3 et 5 à 7 ou utilisation selon l'une des revendications 4 à 7, caractérisé(e) en ce que le diagnostic de la résistance comprend, sur un échantillon de liquide d'un organisme humain, une étape consistant à isoler des cellules tumorales circulantes, de préférence par un procédé de capture à anticorps multiples, et une étape consistant à déterminer le taux d'expression de la protéine de l'isoforme 3 d'ERCC1 et/ou de l'ARNm de l'isoforme 3 d'ERCC1 dans les cellules tumorales circulantes isolées.

9. Utilisation de la protéine de l'isoforme 3 d'ERCC1 selon la revendication 8, utilisation de l'ARNm de l'isoforme 3 d'ERCC1 selon la revendication 8, procédé selon la revendication 8 ou utilisation selon la revendication 8, caractérisé(e) en ce que l'étape consistant à isoler des cellules tumorales circulantes comprend ou consiste en les étapes consistant à
a) mélanger un échantillon de liquide avec une combinaison prédéterminée d'au moins deux anticorps et/ou dérivés d'anticorps possédant une affinité de liaison aux cellules tumorales circulantes ; et
b) isoler des complexes entre i) les anticorps et/ou dérivés d'anticorps et ii) les cellules tumorales circulantes, à partir de l'échantillon.

10. Utilisation de la protéine de l'isoforme 3 d'ERCC1 selon la revendication 9, utilisation de l'ARNm de l'isoforme 3 d'ERCC1 selon la revendication 9, procédé selon la revendication 9 ou utilisation selon la revendication 9, caractérisé(e) en ce que lesdits anticorps et/ou dérivés d'anticorps sont couplés à
a) au moins une particule magnétique ou paramagnétique et, de préférence, une force magnétique est utilisée pour l'isolement desdits complexes ; et/ou
b) au moins une molécule fluorescente et, de préférence, un tri cellulaire activé par fluorescence est utilisé pour l'isolement desdits complexes.

11. Utilisation de la protéine de l'isoforme 3 d'ERCC1 selon l'une des revendications 9 et 10, utilisation de l'ARNm de l'isoforme 3 d'ERCC1 selon l'une des revendications 9 et 10, procédé selon l'une des revendications 9 et 10 ou utilisation selon l'une des revendications 9 et 10, caractérisé(e) en ce au moins un des deux anticorps et/ou dérivés d'anticorps est sélectionné dans le groupe comprenant ou consistant en GP1.4, MOC-31, Ber-EP-4, HMPV.2, HMEIV.2, 8B6, E29, 10E9 (anti-HER2), 2D3 (anti-EpCAM), un anti-cMET, un anti-EGFR, un anti-cKIT, un anti-IGFR et 131-11741.

12. Utilisation de la protéine de l'isoforme 3 d'ERCC1 selon l'une des revendications 8 à 11, utilisation de l'ARNm de l'isoforme 3 d'ERCC1 selon l'une des revendications 8 à 11, procédé selon l'une des revendications 8 à 11 ou utilisation selon l'une des revendications 8 à 11, caractérisé(e) en ce que le diagnostic de la résistance comprend les étapes consistant à
a) lyser lesdites cellules tumorales circulantes isolées,
b) mesurer la quantité de la protéine de l'isoforme 3 d'ERCC1 et/ou de l'ARNm de l'isoforme 3 d'ERCC1, présente dans les cellules tumorales circulantes isolées lysées, et
c) mesurer la quantité d'au moins un ARNm, qui est présent dans les cellules tumorales circulantes isolées lysées et est différent de l'ARNm de l'isoforme 3 d'ERCC1, où le au moins un ARNm est sélectionné dans le groupe comprenant ou consistant en des ARNm qui sont des marqueurs pour des cellules d'un phénotype épithélial, mésenchymateux ou de cellules souches,
dans laquelle la séquence de l'étape b) et l'étape c) peut être modifiée, et
dans laquelle la mesure à l'étape b) et/ou l'étape c) est de préférence réalisée par RT-PCR, LCR, NASBA, un procédé d'hybridation et/ou un procédé comprenant les étapes de PCR, de digestion du produit de PCR avec des enzymes de restriction en fragments, et d'analyse desdits fragments obtenus.

13. Utilisation de la protéine de l'isoforme 3 d'ERCC1 selon l'une des revendications 1 et 5 à 12, utilisation de l'ARNm de l'isoforme 3 d'ERCC1 selon l'une des revendications 2 et 5 à 12, procédé selon l'une des revendications 3 et 5 à 12 ou utilisation selon l'une des revendications 4 à 12, caractérisé(e) en ce que le diagnostic de la résistance comprend une étape consistant à comparer un taux d'expression mesuré de la protéine de l'isoforme 3 d'ERCC1 et/ou de l'ARNm de l'isoforme 3 d'ERCC1 avec une première valeur prédéterminée, où un taux d'expression mesuré de la protéine de l'isoforme 3 d'ERCC1 et/ou de l'ARNm de l'isoforme 3 d'ERCC1 supérieur à la première valeur prédéterminée indique une résistance audit agent thérapeutique.

14. Utilisation de la protéine de l'isoforme 3 d'ERCC1 selon la revendication 13, utilisation de l'ARNm de l'isoforme 3 d'ERCC1 selon la revendication 13, procédé selon la revendication 13 ou utilisation selon la revendication 13, caractérisé(e) en ce que le diagnostic de la résistance comprend les étapes consistant à
a) comparer un taux d'expression mesuré de la protéine de l'isoforme 3 d'ERCC1 et/ou de l'ARNm de l'isoforme 3 d'ERCC1 avec une première valeur prédéterminée ; et
b) comparer un taux d'expression mesuré d'au moins un ARNm, qui est différent de l'ARNm de l'isoforme 3 d'ERCC1, avec une seconde valeur prédéterminée, où le au moins un ARNm est sélectionné dans le groupe comprenant ou consistant en des ARNm qui sont des marqueurs pour des cellules d'un phénotype épithélial, mésenchymateux ou de cellules souches, et
où un taux d'expression mesuré de ladite protéine de l'isoforme 3 d'ERCC1 et/ou dudit ARNm de l'isoforme 3 d'ERCC1 supérieur à la première valeur prédéterminée et un taux d'expression mesuré dudit au moins un ARNm, qui est différent de l'ARNm de l'isoforme 3 d'ERCC1, supérieur à la seconde valeur prédéterminée, indique une résistance audit agent thérapeutique.
